# EUROPEAN PATENT SPECIFICATION

(11) **EP 0 885 910 B1**
(45) Date of publication and mention of the grant of the patent: **01.03.2006**
(21) Application number: 97310544.8
(22) Date of filing: 23.12.1997
(51) Int. Cl.: C08G 59/50, C07C 209/82, C07C 211/09

(54) **Aliphatic polyamines as curing agents for epoxy resins**
Aliphatische Polyamine als Härter für Epoxidharze
Polyamines aliphatiques commes durcisseurs pour des resines epoxydes

(30) Priority: 20.06.1997 JP 16437997
(43) Date of publication of application: 23.12.1998
(73) Proprietor: TORAY INDUSTRIES, INC., Tokyo 103-8666 (JP)
(72) Inventor: Oosedo, Hiroki, Iyo-gun, Ehime 791-31 (JP); Aoki, Ikuo, Iyo-gun, Ehime 791-31 (JP); Kouchi, Shinji, Ehime 790 (JP)
(74) Representative: Coleiro, Raymond

(56) References cited:
- US-A- 4 147 717
- DATABASE WPI Section Ch, Week 9641 Derwent Publications Ltd., London, GB; Class A21, AN 96-408474 XP002900087 & JP 08 198950 A (MITSUI TOATSU CHEM. INC.), 6 August 1996

## Description

The present invention concerns an aliphatic polyamine material and a method of producing a reduced odour liquid aliphatic polyamine. More particularly, the invention relates to the use of an aliphatic polyamine material as a curing agent for an epoxy resin in an epoxy resin composition, particularly, a two-pack type epoxy resin composition, which is used, for example, as an adhesive, a coating material, a molding material or a matrix resin of composite material.

Conventional two-pack epoxy resin compositions, wherein an epoxy resin and a curing agent are mixed immediately before use, are widely used, for example, as adhesives, coating materials, primers, sealants, molding materials and matrix resins of fiber-reinforced composite materials, due to various advantages of the two-pack epoxy resin compositions, in that, for example, they are curable at ambient temperature, they can be used without any solvent, no gas production occurs during curing and they provide products having excellent properties after being cured.

Aliphatic polyamines, excellent in curability at normal temperatures, are widely used as a main component of the curing agent of two-pack epoxy resin compositions.

However, many aliphatic polyamines have strong ammoniacal odours, which often presents problems in indoor use.

If a conventional two-type epoxy resin composition is used as an adhesive, a coating material, a primer, a sealant or a matrix resin of a fiber-reinforced composite material, in rebuilding or repair of an interior of a building which has already been built and used, it is necessary to perform the rebuilding or repair when people are not in the building, for example, during night hours or holidays, or to request people to stay away from the building during the rebuilding or repair, considering the effect of the strong odour on people. Therefore, the strong foul odor of aliphatic polyamine often causes an increase in the period allowed for a rebuilding or repairing operation.

JP-A-8-198950 discloses the use of BCHA-acrylonitrile adducts for curing epoxy resins which are stated to be substantially free of an amine-like odour.

The present invention addresses the problem of reducing the odour of a liquid comprising an aliphatic polyamine for use as curing agent to a very low level.

Accordingly, in a first aspect the present invention provides the use of an aliphatic polyamine material, which is a liquid comprising an aliphatic polyamine capable of liberating ammonia in the presence of oxygen and from which volatile odour components have been removed to provide a reduced odour liquid aliphatic polyamine, the reduced odour liquid polyamine being sealed in an air-tight container in the absence of oxygen, and having a characteristic such that when the liquid is air-tightly sealed in the container together with a gas containing no oxygen and kept at 20°C, the ammonia concentration in the gas in an equilibrium is at most 100 ppm by volume, as a curing agent for an epoxy resin, which curing agent is suitable for indoor use.

In a second aspect the present invention provides a two-pack epoxy resin composition comprising an epoxy resin, an aliphatic polyamine material which is a liquid comprising an aliphatic polyamine capable of liberating ammonia in the presence of oxygen and from which volatile odour components have been removed to provide a reduced odour liquid aliphatic polyamine, the reduced odour liquid aliphatic polyamine being sealed in an air-tight container in the absence of oxygen and having a characteristic such that when the reduced odour liquid is air-tightly sealed in the container together with a gas containing no oxygen and kept at 20°C, the ammonia concentration in the gas in an equilibrium is at most 100 ppm by volume, and an adsorbent having adsorption capacity for volatile odour components.

In a method of producing an aliphatic polyamine material of the present invention, dissolved volatile odour components are removed from a liquid comprising an aliphatic polyamine, and the liquid is then stored in an air-tight container with substantially no oxygen present therein.

The liquid may be present in a container together with a gas or may fill the entire container.

Embodiments of the invention will now be described in more detail. Regarding the odour of aliphatic polyamines, our research has surprisingly revealed the following:
(A) The problematic odour is not caused by the aliphatic polyamine itself but caused by highly volatile impurities such as ammonia. Removal of these impurities eliminates the odour.
(B) Even if such volatile odour components are removed before storage, storage in the presence of oxygen allows gradual production of volatile odour components and, therefore, production of foul odour. Storage substantially

in the absence of oxygen does not allow production of foul odor.

According tc the invention, the liquid comprises an aliphatic polyamine. The liquid material may be formed from a single kind of aliphatic polyamine alone, or a composition comprising different kinds of aliphatic polyamine, or a composition containing one or more kinds of aliphatic polyamine as a main component (i.e. present in an amount by weight no less than the amount of any other component and preferably at least 50% by weight of the total composition) and further containing other components.

If the liquid material comprises a single kind of aliphatic polyamine, only removal of the volatile odor components such as ammonia from the aliphatic polyamine, is necessary. Normally, oxygen is also removed by the process of removing volatile odor components.

If the liquid comprising aliphatic polyamine according to the invention is formed from a mixture of a plurality of materials, either of two methods may be employed: a method in which after volatile odor components are removed from each material, the materials are mixed in an atmosphere containing substantially no oxygen, or n method in which atter the materials are mixed, volatile odor components are removed from the mixture.

Examples of a process for removing volatile odor components from the liquid comprising aliphatic polyamine include a vacuum degasification in which a liquid to be processed is subjected to a reduced pressure in a sealed container for a predetermined length of time, and then brought back to an atmospheric pressure using a gas that contains substantially no oxygen. The temperature for the pressure reducing process may be an ambient temperature or an appropriately elevated temperature. Further, the liquid may be stirred or may not be stirred during the pressure reducing procces.

In the vacuum degasification, a gas containing substantially no oxygen may be passed through the liquid comprising the aliphatic polyamine. This method is effective to improve the efficiency in removing volatile odor components. The method advantageotsly reduces the processing time particularly if a large amount of liquid is to be treated. Furthermore, a high-degree vacuum is not required in the pressure reducing process.

Another example of the process for removing volatile odor components from the liquid comprising the aliphatic polyamine is an aeration process using a gas containing substantially no oxygen. In the process, a gas containing substantially no oxygen is passed through the liquid al. an atmospheric pressure for a predetermined length of time. The temperature for the aeration process may be an ambient temperature or an appropriately elevated temperature. Further, the liquid may be stirred or may not be stirred during the pressure reducing process.

Although the aforementioned two methods are effective to remove volatile odor components from the liquid containing the aliphatic polyamines, a distillation method may also be used. Either of the following two distillation methods may be used: a method in which distillation is performed in an atmosphere containing substantially no oxygen; and a method in which distillation is performed at a reduced pressure, and the liquid is brought back to an atmospheric pressure using a gas containing substantially no oxygen. However, application of the distillation method may not be easy in the case of a mixture wherein the boiling points of components are considerably different.

A liquid comprising an aliphatic polyamine prepared by a method as described above is sealed into an air-tight container, with substantially no oxygen contained therein. An aliphatic polyamine material is thereby produced. Therefore, in the aliphatic polyamine material of the invention, a liquid comprised of an aliphatic polyamine is sealed in an air-tight container.

In a method of sealing a liquid in an air-tight container with substantially no oxygen contained therein, a liquid containing an aliphatic polyamine is placed or injected, together with a gas containing substantially no oxygen, into an air-tight container, and then sealed therein.

In another method that may be used, the capacity of an air-tight container is filled with a liquid comprising aliphatic polyamine so that a space for gas is substantially eliminated.

In the invention, any air-tight container may be used as long as it has good sealing characteristic and low oxygen permeability. For example, a jar, a bottle, a can, a tank, or an ampoule formed of a metal, glass or plastics material. A squeeze-type tube or bag, or an extruding container comprised of a piston and a cylinder, may be preferred since such a container allows a desired amount of the liquid to be used without substantially causing the content of the container to be exposed to the external air.

The atmosphere containing substantially no oxygen, which is used in the processes described above, is preferably a gas in which the oxygen concentration is at most 1 vol.%. More specifically, commercially available nitrogen or argon may preferably be used.

The aliphatic polyamine according to the invention refers to a compound having, in its molecule, a plurality of amino groups which connect carbons having sp³ hybrid orbitals. If these conditions are satisfied, an amine having an aromatic ring in its molecule may be regarded as an aliphatic polyamine having an aromatic substituent. That is, such an amine having an aromatic ring is included in the aliphatic polyamine according to the invention.

Examples of the aliphatic polyamine present in or consisting of aliphatic polyamine material according to the invention include: hexamethylenediamine, 2,5-dimethyl-2,5-hexanediamine. 2,2,4-trimethylhexamethylenediamine, diethylenetriamine, triethylenetetramine, tetraethylenepentamine, pentaethylenehexamine, 4-aminomethyloctanethylenediamine, 3,3'-iminobis(propylamine), 3,3'-methyliminobis(propylamine), bis (3-aminopropyl)ether, 1,2-bis(3-aminopropyloxy)ethane, menthanediamine , isophoroneciamine, bisaminomethylnorbornane, bis(4 aminocyclohexyl)methane, bis(4-amino-3-methylcyclchexyl)methane, 1,3-diaminocyclohexane, 3,9-bis(3-aminopropyl)-2,4,8,10-tetraoxaspiro[5,5] -undecane. Examples of the aliphatic polyamine having an aromatic substituent group include, for example, m-xylylene diamine and tetrachloro-p-xylylene diamine.

Polyamideamines obtained through reactions between polymerized fatty acids (polyfunctional tatty acid mixture containing dimers of unsaturated fatty acids such as linolic acid) and polyamines, such as ethylenediamine, diethylenetriamine, triethylenetetramine, tetraethylenepentamine and pentaethylenehexmine, may suitably be used.

Furthermore, compounds obtained by introducing aminoalkyl groups into the two terminals of a polyether, such as polyethylene glycol and polypropylene glycol and compounds obtained by introducing aminoalkyl groups into the two terminals of a silicone compounds, such as polydimethylsiloxane and polymethylphenylsiloxane may also be suitably used.

According to the invention, the liquid comprising aliphatic polyamine may also contain amines other than aliphatic polyamine, for example, imidazoles, or curing agents other than amines, for example, polymercaptans, which are dissolved or dispersed therein.

According to the invention, the liquid comprising aliphatic polyamine may further contain a dissolved or dispersed phenol compound as a curing accelerator for the aliphatic polyamine. Preferable examples of the phenol compounds include diisopropylphenol and nonylphenol.

According to the invention, the liquid comprising aliphatic polyamine may further contain plasticizers, dyes, organic or inorganic particles such as organic pigments, carbon black and silica, high molecular weight at compounds, antioxidants, ultraviolet absorbents, coupling agento and surfactants, which are dissolved or dispersed therein.

To evaluate the low-odor characteristic of the liquid comprising aliphatic polyamine according co che invention, the ammonia concentration in a gas that is in an equilibrium state with the liquid comprising aliphatic polyamine is measured, since ammonia has the greatest contribution to the odor of the liquid. The ammonia concentration in the gas indicates the upper limit of odor that can occur during operation using the liquid comprised of aliphatic polyamine. Due to the Henry's law, the ammonia concentration in the gas is proportional to the ammonia concentration in the liquid.

The ammonia concentration according to the invention reters to an ammonia concentration in a gas which is in equilibrium with the liquid comprised of aliphatic polyamine. The ammonia concentration according to the invention is measured by the following method.

A sealed container in which air and the liquid comprising the aliphatic polyamine are contained is kept at 20°C for 4 hours, so that equilibrium is attained.

Thereafter, an end of an ammonia detector tube is inserted into the container through a small opening that does not disturb the ammonia concentration therein, the gas is drawn and color change of the detector tube is read.

A commercially available ammonia detector tube may be used. Since there are several types of ammonia detector tubes with difference detectable concentration ranges, a type suitable to the specimen must be used.

Examples of commercially available ammonia detector tubes include produces by Gastec Kabushiki Gaisha. Gastec provides various types of ammonia detector tubes with different ammonia detectable concentration ranges as follows, and a suitable type can be selected from them.

| | |
|---|---|
| Ammonia No. 3L: | 0.5-60 ppm |
| Ammonia No. 3La: | 2.5-200 ppm |
| Ammonia No. 3M: | 10-1000 ppm |
| Ammonia No. 3HM: | 0.05-3.52% |
| Ammonia No. 3H: | 0.2-32% |

If the ammonia concentration in the gas in equilibrium with the liquid comprising the aliphatic polyamine is 100 ppn or less, irritation will not become problem although there may be olight ammoniacal odor. Therefore, an ammonia concentration of 100 ppm is preferable. An ammonia concentration of 10 ppm is more preferable since there is no substantial ammoniacal odor.

According to the invention, if the liquid comprising the aliphatic polyamine is used as a curing agent of an epoxy resin, the sealed package of the aliphatic polyamine material produced according to the invention is open immediately before it is used. The content is mixed with the epoxy resin to form an epoxy resin composition, which is than actually used. In the aliphatic polyamine material produced according to the invention, the liquid comprising the aliphatic polyamine is made substantially free from volatile odor components and sealed in an air-tight container with substantially no oxygen contained. Therefore, no odor will be produced by oxidation of aliphatic polyamine even during a long period of storage, so that no odor will be released when the container is opened.

The epoxy resin refers to an epoxy compound having a plurality of epoxy groups in a molecule.

Examples of the epoxy resin to be used in the invention include bisphenol A-type epoxy resin, bisphenol F-type epoxy resin, bisphenol S-type epoxy resin, biphenyl-type epoxy resin, naphthalene-type epoxy resin, novolac-type epoxy resin, epoxy resin having a fluorene skeleton, epoxy resin derived from copolymer of a phenol compound and dicyclopentadiene, glycidyl ether-type epoxy resin such as diglycidylresorcinol, tetrakis(glycidyloxyphenyl)ethane and tris(glycidyloxyphenyl)methane, glycidylamine-type epoxy resins such as tetraglycidyldiaminodiphenylmethana, triglycidylaminophenol, triglycidylaminocresol and tetraglycidylxylenediamine, alicyclic epoxy resins such as vinylcyclohexenediepoxide and mixtures of any of these compounds .

It is also possible to compound into the epoxy resin composition a compound having one epoxy group in its molecule as a reactive diluent. Examples of the compounds having one epoxy group in its molecule include phenyl glycidyl ather, butyl glycidyl ether, allyl glycidyl ether, styrene oxide and octylene oxide.

It is also possible to compound into the epoxy rein composition a compound, other than epoxy compounds, which reacts with amines. Examples of the component that reacts with amines include isocyanates such as hexamethylene diisocyanate and tolylene diisocyanate, and further, α, β-unsaturated carbonyl compounds that undergo the Michael addition reactions with amines.

The epoxy resin composition may further contain other components, for example, plasticizers, dyes, organic or inorganic particles such as organic pigments, carbon black, silica, alumina and clay minerals, macromolecular compounds, antioxidants, ultraviolet absorbents, coupling agents and surfactants.

Components having adsorption capacity, such as silica, alumina, clay minerals, may preferably compounded since such component will adsorb small amounts of volatile odor components that remain even after the volatile odor component removing process.

The epoxy resin composition may be used as an adhesive, a coating material,a primer, a sealant, a molding material or a matrix resin of a fiber-reinforced composite material .

To use the epoxy resin composition as a matrix resin of a fiber-reinforced composite material, a hand lay-up method may be employed wherein a sheet-formed reinforcing fiber is impregnated with an epoxy resin composition, and laminated, and then cured at a room temperature or a temperature higher Lhan the room temperature. Examples of the reinforcing fibers to be used include carbon fiber, glass fiber and aramid fiber. The fibers may be formed into a sheet-like form, such as a woven fabric, a mat and a unidirectional sheet.

The hand lay-up method is used in production of component parts of a bath tub, a tank, a hull, a motor vehicle, and repair or reinforcement of fiber-reinforced composite materials and concrete structures.

Examples of the method for using the epoxy resin composition as a matrix resin of a fiber-reinforced composite material further include a filament winding method and a resin transfer molding method.

In the aliphatic polyamine material produced according to the invention, the liquid comprising the aliphatic polyamine is made substantially free from volatile odor components and sealed in an air-tight container with substantially no oxygen contained. Therefore, no odor will be produced by oxidation of aliphatic polyamine even during a long period of storage, so that no odor will be released when the container is opened. Therefore, if it is used as a curing agent in an epoxy rein composition that is used as an adhesive, a coating material, a primer, a sealant , a matrix of a fiber-reinforced composite material for repair or reinforcement; in an indoor operation, more particularly, in rebuilding or repair of an interior of a building which is in use, the aliphatic polyamine material of the invention does not give irritating odor or uneasy feeling to people who use the building and, therefore, can suitably be used in such a situation.

Embodiments of the invention will now be further described in more detail with reference to Examples.

### EXAMPLE 1

Two specimens were prepared in each of which 50 g of commercially obtained bis(aminomethyl)norbornane (by Mitsui Tostsu Fine Chemicals, Inc.) was placed in a flask having a capacity of about 200 cm³ and equipped with a three-way cock. Strong ammonia odor was smelled from the specimens. The ammonia concentration detected by an ammonia detector tube (Ammonia No. 3HM by Gastec) was 1400 ppm.

One of the two specimens was subjected to degasification for 10 minutes under a reduced pressure of about 200 Pa using a vacuum pump. After the degasification, no ammonia odor as before the degasification was not smelled from the specimen. The ammonia concentration detected by an ammonia detector tube (Ammonia No. 3L by Gastec) was 2 ppm.

The other specimen was also subjected to degasification for 10 minutes under a reduced pressure of about 200 Pa using a vacuum pump. Subsequently, nitrogen gas was introduced into the container to resume an atomospheric pressure. After the three-way cock was air-tightly closed, the specimen was left in an oven at 50°C for 20 hours. The container was then cooled to 20°C for cetection of ammonia concentration. The ammonia concentration detected was 4 ppm.

### EXAMPLE 2

50 q of bis(aminomethyl)norbornane (by Mitsui Toatsu Fine Chemicals, Inc.) the same as used in Example 1 was placed in a flask having a capacity of about 200 cm³. Nitrogen qas was passed through the liquid at a flow rate of 100 cm³/minute for an hour using a glass tube whose end was placed in the liquid. After the treatment, no ammonia odor as before the treatment was not smelled. The ammonia concentration in the container was detected as 8 ppm. 30 cm³ of the treated bis(aminomethyl)norbornane was collected by a syringe, and injected into a flask having a capacity of about 100 cm³ and equipped with a three-way cock, whose interior atmosphere had been replaced with nitrogen gas. After the cock was air-tightly closed, the specimen was left in an oven at 50°c for 20 hours. The specimen was cooled to 20°C for detection of ammonia concentration. The ammonia concentration detected was 2 ppm.

### EXAMPLE 3

Bis(aminomethyl)norbornane was degasified as in Example 1 and brought back to an atomospheric pressure using nitrogen gas. Under a nitrogen gas stream, a specimen of 10 cm³ was measured into a glass syringe. After air inside the syringe was discharged by pushing the piston while holding and directing the syringe upward, the tip end of the syringe was sealed by a rubber cap. The specimen was left in an oven at 50°C for 20 hours. After the specimen was cooled to 20°C, the cap was removed and the specimen was placed into another container to check the odor. No ammonia odor was detected. The ammonia concentration detected was 2 ppm.

### COMPARATIVE EXAMPLE 1

Bis(aminomethyl)norbornane was degasified as in Example 1 and brought back to an atomospheric pressure using air. After the three-way cock was closed to air-tightly seal the container, the container was left in an oven at 50°C for 20 hours. After the container was cooled to 20°C, the three-way cock was opened to check the odor. A acrong ammonia odor prevailed. The ammonia concentration detected by an ammonia detector tube (Ammonia NO. HM by Gastec) was 2000 ppm.

### EXAMPLE 4

25 g of bis(aminomethyl)norbornane (by Mitsui Toatsu Fine Chemicals, Inc.), 25 g of isophoronediamine (Wako Pure Chemical Indrustries, Ltd.) and 2 g of Aerosil 380 (Nippon Aerosil Co., Ltd.) were placed in flask having a capacity of about 200 cm³ and equipped with a three-way cock. After the mixture was stirred for 20 minutes by a magnetic stirrer, the mixture was degasified at a reduced pressure of about 200 Pa for 10 minutes using a vacuum pump. The degasified specimen was brought back to an atomospheric pressure using nitrogen gas. After the three-way cock was closed to air-tightly seal the container, the container was left in an oven at 50°C for 20 hours. After the container was cooled to 20°C, the cock was opened to check the odor. No ammonia odor was detected. The ammonia concentration detected was 5 ppn.

### EXAMPLE 5

1 kg of bis(aminomethyl)norbornane (by Mitsui Toatsu Fine Chemicals, Inc.) was placed into a cylindrical separable flask equipped with a gas introducing tube and a stirring blade. While nitrogen gas was gradually introduced into bis(aminomethyl)norbornane through the gas introducing tube and the liquid was stirred, the pressure was reduced to about 40C Pa for 2 hours using an aspirator. No ammonia odor was detected from the treated liquid. The ammonia concentration detected was 5 ppm.

450 cm³ of the treated bis(aminomethyl)norbornane was poured into a metallic can having a capacity of about 500 cm³, with nitrogen gas blown into the can. After the can was immediately lidded air-tightly, the specimen was Left in an over at 50°C for 20 hours. After the container was cooled to 20°C, the ammonia concentration was detected. The ammonia concentration detected was 8 ppm. No ammonia odor was detected.

### EXAMPLE 6

Two specimens were prepared in each of which 50 g of commercially obtained triethylenetetramine (by ACI Japan Ltd.) was placed in a flask having a capacity of about 200 cm³ and equipped with a three-way cock. A strong ammonia odor from the specimens prevailed. The ammonia concentration detected was 2500 ppm.

One of the two specimens was subjected to degasification for 10 minutes under a reduced pressure of about 200 Pa using a vacuum pump.. After the degasification, the ammonia odor which prevailed before the degasification no longer exuded from the specimen.
The ammonia concentration detected was 40 ppm.

The other specimen was also subjected to degasification for 10 minutes under a reduced pressure of about 200 Pa using a vacuum pump. Subsequently, nitrogen gas was introduced into the container to resume an atomospheric pressure. After the three-way cock was air-tightly closed, the specimen was left in an oven at 50°C for 20 hours . The container was then cooled to 20°C for detection of ammonia concentration. The ammonia concentration detected was 70 ppm.

### EXAMPLE 7

Two specimens were prepared in each of which 50 g of commercially obtained diethylenetriamine (by ACI Japan Ltd.) and 40 g of bis(4-amino-3-methylcyclohexyl)methane (by ACI Japan Ltd.) were placed in a flask having a capacity of about 200 cm³ and equipped with a three-way cock. A strong ammonia odor from the specimens prevailed. The ammonia concentration detected was 1000 ppm.

One of the two specimens was subjected to degasification for 10 minutes under a reduced pressure of about 200 Pa using a vacuum pump. After the degasification, the ammonia odor which prevailed before the degasification no longer exuded from the specimen. The ammonia concentration detected was 16 ppm.

The other specimen was also subjected to degasification for 10 minutes under a reduced pressure of about 200 Pa using a vacuum pump. Subsequently, nitrogen gas was introduced into the container to resume an atomospheric pressure. After the three-way cock was air-tightly closed, the specimen was left in an oven at 50°C for 20 hours. The container was then cooled to 20°C for detection of ammonia concentration. The ammonia concentration detected was 20 ppm.

### EXAMPLE 8

50 g of m-xylylene diamine (by Mitsubishi Gas Chemical Company Inc.) was weighed in a flask having a capacity of about 200 cm³. A strong ammonia odor from the specimen prevailed. The ammonia concentration detected was 500 ppm.

Subsequently, the specimen was subjected to distillation under a reduced pressure while nitrogen was passed through it. A distillation fraction of a boiling point of 80-95°C/l mmHg was obtained. No ammonia odor was detected from this liquid. The ammonia concentration detected was 60 ppm.

Further, 30 cm³ of m xylylene diamine, after the distillation, was taken into a cyringe, and injected into a flask having a capacity of about 100 cm³ and equipped with a three-way cock, whose interior atmosphere had been replaced with nitrogen gas. After leaving the specimen in an oven at 50°C for 20 hours, the specimen was cooled to 20°C for detection of ammonia concentration. The ammonia concentration detected was 80 ppm.

## Claims

1. Use of an aliphatic polyamine material, which is a liquid comprising an aliphatic polyamine capable of liberating ammonia in the presence of oxygen and from which volatile odour components have been removed to provide a reduced odour liquid aliphatic polyamine, the reduced odour liquid polyamine being sealed in an air-tight container in the absence of oxygen, and having a characteristic such that when the liquid is air-tightly sealed in the container together with a gas containing no oxygen and kept at 20°C, the ammonia concentration in the gas in an equilibrium is at most 100 ppm by volume, as a curing agent for an epoxy resin, which curing agent is suitable for indoor use.

2. Use according to claim 1, wherein the reduced odour aliphatic polyamine material is stored in a container such that the container is entirely filled with the liquid or contains the liquid together with a gas containing no oxygen.

3. Use according to claim 1 or claim 2, wherein the liquid has a characteristic such that when it is sealed in the container together with the gas and kept at 20°C, the ammonia concentration in the gas in an equilibrium is at most 10 ppm by volume.

4. Use according to any one of claims 1 to 3, wherein the reduced odour aliphatic polyamine material is mixed with the epoxy resin and the mixture of the epoxy resin is used in reforming or repairing the interior of a building.

5. Use according to claim 4, wherein the mixture of epoxy resin and reduced odour aliphatic polyamine material is used as a matrix resin of a fiber-reinforced composite material, or as an adhesive, or as a coating material, or as a primer, or as a sealant.

6. Use according to claim 5, wherein the said mixture is used as a matrix resin of a fiber-reinforced composite material.

7. Use according to any preceding claim, wherein the reduced odour aliphatic polyamine material includes diethylenetriamine, triethylenetetramine, isophoronediamine, bisaminomethylnorbornane, bis(4-amino-3-methylcyclohexyl)methane, or m-xylylene diamine.

8. A two-pack epoxy resin composition comprising an epoxy resin, an aliphatic polyamine material which is a liquid comprising an aliphatic polyamine capable of liberating ammonia in the presence of oxygen and from which volatile odour components have been removed to provide a reduced odour liquid aliphatic polyamine, the reduced odour liquid aliphatic polyamine being sealed in an air-tight container in the absence of oxygen and having a characteristic such that when the reduced odour liquid is air-tightly sealed in the container together with a gas containing no oxygen and kept at 20°C, the ammonia concentration in the gas in an equilibrium is at most 100 ppm by volume, and an adsorbent having adsorption capacity for volatile odour components.

9. A two-pack epoxy resin composition according to claim 8, wherein the adsorbent is selected from silica, alumina and clay minerals.

10. A two-pack epoxy resin composition according to claim 8 or 9 wherein the reduced odour liquid aliphatic polyamine material entirely fills the container.

11. A two-pack epoxy resin composition according to claim 8 or 9, wherein the reduced odour liquid aliphatic polyamine material is air-tightly sealed in the container together with a gas.

12. A two-pack epoxy resin composition according to any one of claims 8 to 11, wherein the reduced odour aliphatic polyamine material is selected from diethylenetriamine, triethylenetetramine, isophoronediamine, bisaminomethylnorbornane, bis(4-amino-3-methylcyclohexyl)methane, and m-xylylene diamine.

## Revendications

1. Utilisation d'une matière sous forme de polyamine aliphatique, laquelle est un liquide comprenant une polyamine aliphatique capable de libérer de l'ammoniac en présence d'oxygène et dont les éléments constitutifs d'odeur volatile ont été retirés pour fournir une polyamine aliphatique liquide d'odeur réduite, la polyamine liquide d'odeur réduite étant enfermée dans un récipient étanche à l'air en l'absence d'oxygène, et présentant une caractéristique telle que, lorsque le liquide est enfermé de manière étanche à l'air dans le récipient en même temps qu'un gaz ne contenant pas d'oxygène et maintenu à 20°C, la concentration en ammoniac du gaz dans un équilibre est au plus de 100 ppm par volume, en tant qu'agent de polymérisation pour une résine époxy, lequel agent de polymérisation est approprié pour une utilisation à l'intérieur d'un local.

2. Utilisation selon la revendication 1, dans laquelle la matière sous forme de polyamine aliphatique d'odeur réduite est stockée dans un récipient de telle manière que le récipient soit entièrement rempli avec le liquide ou contienne le liquide en même temps qu'un gaz ne contenant pas d'oxygène.

3. Utilisation selon la revendication 1 ou la revendication 2, dans laquelle le liquide présente une caractéristique telle que, lorsqu'il est enfermé dans le récipient en même temps que le gaz et maintenu à 20°C, la concentration en ammoniac du gaz dans un équilibre est au plus de 10 ppm par volume.

4. Utilisation selon l'une quelconque des revendications 1 à 3, dans laquelle la matière sous forme de polyamine aliphatique d'odeur réduite est mélangée avec la résine époxy et le mélange de résine époxy est utilisé dans le refaçonnage ou la réparation de l'intérieur d'un bâtiment.

5. Utilisation selon la revendication 4, dans laquelle le mélange de résine époxy et de matière sous forme de polyamine aliphatique d'odeur réduite est utilisé en tant que résine de matrice d'une matière composite à renfort de fibres, ou en tant que milieu adhésif, ou en tant que matière de revêtement, ou en tant que couche de fond, ou en tant que matériau d'étanchéité.

6. Utilisation selon la revendication 5, dans laquelle ledit mélange est utilisé en tant que résine de matrice d'une matière composite à renfort de fibres.

7. Utilisation selon une quelconque revendication précédente, dans laquelle la matière sous forme de polyamine aliphatique d'odeur réduite comprend de la diéthylènetriamine, de la triéthylènetétramine, de l'isophoronediamine, du bisaminométhylnorbornane, du bis(4-amino-3-méthylcyclohexyl)méthane, ou de la m-xylylène diamine.

8. Une composition de résine époxy à deux composants comprenant une résine époxy, une matière sous forme de polyamine aliphatique qui est un liquide comprenant une polyamine aliphatique capable de libérer de l'ammoniac en présence d'oxygène et dont les éléments constitutifs d'odeur volatile ont été retirés pour fournir une polyamine aliphatique liquide d'odeur réduite, la polyamine aliphatique liquide d'odeur réduite étant enfermée dans un récipient étanche à l'air en l'absence d'oxygène et présentant une caractéristique telle que, lorsque le liquide d'odeur réduite est enfermé de manière étanche dans le récipient en même temps qu'un gaz ne contenant pas d'oxygène et maintenu à 20°C, la concentration en ammoniac du gaz dans un équilibre est au plus de 100 ppm par volume, et un adsorbant ayant une capacité d'adsorption pour des éléments constitutifs d'odeur volatile.

9. Une composition de résine époxy à deux composants selon la revendication 8, dans laquelle l'adsorbant est sélectionné à partir de minéraux à base de silice, d'alumine et d'argile.

10. Une composition de résine époxy à deux composants selon la revendication 8 ou 9 dans laquelle la matière sous forme de polyamine aliphatique liquide d'odeur réduite remplit entièrement le récipient.

11. Une composition de résine époxy à deux composants selon la revendication 8 ou 9, dans laquelle la matière sous forme de polyamine aliphatique liquide d'odeur réduite est enfermée de manière étanche à l'air dans le récipient en même temps qu'un gaz.

12. Une composition de résine époxy à deux composants selon l'une quelconque des revendications 8 à 11, dans laquelle la matière sous forme de polyamine aliphatique d'odeur réduite est sélectionnée à partir de la diéthylènetriamine, la triéthylènetétramine, l'isophoronediamine, le bisaminométhylnorbornane, le bis(4-amino-3-méthylcyclohexyl)méthane, et la m-xylylène diamine.

## Patentansprüche

1. Verwendung eines aliphatischen Polyaminmaterials, das eine Flüssigkeit ist, die ein aliphatisches Polyamin umfasst, das zur Freisetzung von Ammoniak in Gegenwart von Sauerstoff in der Lage ist, und von dem flüchtige Geruchskomponenten entfernt wurden, um ein flüssiges aliphatisches Polyamin mit verringertem Geruch bereitzustellen, wobei das flüssige Polyamin mit verringertem Geruch in Abwesenheit von Sauerstoff in einem luftdichten Behälter eingeschlossen ist und das Merkmal aufweist, dass, wenn die Flüssigkeit zusammen mit einem keinen Sauerstoff enthaltenden Gas luftdicht in dem Behälter eingeschlossen ist und bei 20 °C gehalten wird, die Ammoniakkonzentration im Gas im Gleichgewicht höchstens 100 Vol.-ppm beträgt, als Härter für ein Epoxidharz, wobei der Härter zur Innenraumanwendung geeignet ist.

2. Verwendung nach Anspruch 1, worin das aliphatische Polyaminmaterial mit verringertem Geruch so in einem Behälter gelagert wird, dass der Behälter vollkommen mit der Flüssigkeit angefüllt ist oder die Flüssigkeit zusammen mit einem keinen Sauerstoff enthaltenden Gas enthält.

3. Verwendung nach Anspruch 1 oder Anspruch 2, worin die Flüssigkeit das Merkmal aufweist, dass, wenn die Flüssigkeit zusammen mit dem Gas luftdicht in dem Behälter eingeschlossen ist und bei 20 °C gehalten wird, die Ammoniakkonzentration im Gas im Gleichgewicht höchstens 10 Vol.-ppm beträgt.

4. Verwendung nach einem der Ansprüche 1 bis 3, worin das aliphatische Polyaminmaterial mit verringertem Geruch mit dem Epoxidharz vermischt wird und das Epoxidharzgemisch verwendet wird, um den Innenraum eines Gebäudes umzugestalten oder zu reparieren.

5. Verwendung nach Anspruch 4, worin das Gemisch aus Epoxidharz und aliphatischem Polyaminmaterial mit verringertem Geruch als Matrixharz eines faserverstärkten Verbundmaterials, als Kleber, als Beschichtungsmaterial, als Primer oder als Dichtungsmittel verwendet wird.

6. Verwendung nach Anspruch 5, worin das Gemisch als Matrixharz eines faserverstärkten Verbundmaterials verwendet wird.

7. Verwendung nach einem der vorangegangenen Ansprüche, worin das aliphatische Polyaminmaterial mit verringertem Geruch Diethylentriamin, Triethylentetramin, Isophorondiamin, Bisaminomethylnorbornan, Bis-(4-amino-3-methylcyclohexyl)methan oder m-Xylylendiamin umfasst.

8. Zweikomponenten-Epoxidharzzusammensetzung, umfassend ein Epoxidharz, ein aliphatisches Polyaminmaterial, das eine Flüssigkeit ist, die ein aliphatisches Polyamin umfasst, das zur Freisetzung von Ammoniak in Gegenwart von Sauerstoff in der Lage ist, und von dem flüchtige Geruchskomponenten entfernt wurden, um ein flüssiges aliphatisches Polyamin mit verringertem Geruch bereitzustellen, wobei das flüssige aliphatische Polyamin mit verringertem Geruch in Abwesenheit von Sauerstoff in einem luftdichten Behälter eingeschlossen ist und das Merkmal aufweist, dass, wenn die Flüssigkeit mit verringertem Geruch zusammen mit einem keinen Sauerstoff enthaltenden Gas luftdicht in dem Behälter eingeschlossen ist und bei 20 °C gehalten wird, die Ammoniakkonzentration im Gas im Gleichgewicht höchstens 100 Vol.-ppm beträgt, und ein Adsorbens mit Adsorptionsfähigkeit für flüchtige Geruchskomponenten.

9. Zweikomponenten-Epoxidharzzusammensetzung nach Anspruch 8, worin das Adsorbens aus Silica, Aluminiumoxid und Tonmineralien ausgewählt ist.

10. Zweikomponenten-Epoxidharzzusammensetzung nach Anspruch 8 oder 9, worin das flüssige aliphatische Polyaminmaterial mit verringertem Geruch den Behälter vollkommen ausfüllt.

11. Zweikomponenten-Epoxidharzzusammensetzung nach Anspruch 8 oder 9, worin das flüssige aliphatische Polyaminmaterial mit verringertem Geruch zusammen mit einem Gas luftdicht im Behälter eingeschlossen ist.

12. Zweikomponenten-Epoxidharzzusammensetzung nach einem der Ansprüche 8 bis 11, worin das aliphatische Polyaminmaterial mit verringertem Geruch aus Diethylentriamin, Triethylentetramin, Isophorondiamin, Bisaminomethylnorbornan, Bis-(4-amino-3-methylcyclohexyl)methan und m-Xylylendiamin ausgewählt ist.
